# EUROPEAN PATENT SPECIFICATION

(11) **EP 3 943 806 B1**
(45) Date of publication and mention of the grant of the patent: **29.03.2023**
(21) Application number: 21187045.6
(22) Date of filing: 21.07.2021
(51) Int. Cl.: F21S 8/02, A61C 1/08, A61G 15/14, A47C 7/74, A61G 10/00

(54) **APPARATUS COMPRISING A SUCTION ASSEMBLY AND AT LEAST ONE ACCESSORY UNIT PROVIDED WITH AN ELECTRONIC AND/OR PNEUMATIC DEVICE**
VORRICHTUNG MIT EINER ABSAUGVORRICHTUNG UND MINDESTENS EINEM ZUBEHÖRTEIL MIT EINER ELEKTRONISCHEN UND/ODER PNEUMATISCHEN VORRICHTUNG
APPAREIL COMPRENANT UN ENSEMBLE D'ASPIRATION ET AU MOINS UNE UNITÉ D'ACCESSOIRE DOTÉE D'UN DISPOSITIF ÉLECTRONIQUE ET/OU PNEUMATIQUE

(30) Priority: 24.07.2020 IT 202000018028
(43) Date of publication of application: 26.01.2022
(73) Proprietor: Piersigilli, Tiziano, 60030 Castelbellino (AN) (IT)
(72) Inventor: Piersigilli, Tiziano, 60030 Castelbellino (AN) (IT)
(74) Representative: Baldi, Claudio

(56) References cited:
- CN-A- 108 852 548
- DE-U1- 29 715 112
- GB-A- 2 334 448

## Description

The present patent application for an industrial invention relates to an apparatus comprising a suction assembly and at least one accessory unit provided with an electronic and/or pneumatic device.

In particular, the present invention has been conceived in the dental field. As it is known, operators and specialists performing a dental service are continuously exposed to pathogenic microorganisms, including viruses and bacteria, which infect the oral cavity and respiratory tract of a patient. In fact, dental procedures involve working in close proximity to the patient's oral cavity, and therefore in close contact with saliva, splashes, and aerosol flows generated by the rotary or piezoelectric tools used during the dental service. Such splashes and aerosol flows are vectors of germs, viruses and bacteria.

In order to reserve their own safety, the operator and/or specialist is required to wear safety devices, such as masks, goggles, and the like.

The advent of the contingent coronavirus pandemic (Covid 19) has demonstrated that these safety devices are no longer appropriate, and therefore new devices have been introduced in dental practices in order to protect the health of both specialists and patients in a proper, safe way.

For this reason, more and more dental practices have adopted suction systems or suction assemblies provided with an inlet that is positioned close to where the practitioner is working, in such a way that splashes, aerosols or the like do not come into contact with the specialist and are sucked up by the suction assembly, thus preserving the health of the specialist and of the assistants.

However, said suction systems or suction assemblies are cumbersome and cannot be easily integrated in the specialized equipment that is already present in the dental practices.

For illustrative purpose, for what specifically refers to the dental sector, said suction assemblies are extremely complex to integrate in a dental unit.

In fact, as it is known, the dental unit is equipped with a plurality of accessory units comprising articulated and/or flexible arms that support monitors, lamps and other electronic devices and comprise conduits for the passage of tubes and/or electrical cables used to power said electropneumatic devices.

In order to integrate a suction assembly in the dental unit, it is necessary that the suction conduit of the suction assembly and the tubes and/or electrical cables all flow into the same support element. Evidently, it is impossible to convey the tubes and/or cables into the aforementioned suction conduit where the sucked air passes, because the cables could be damaged and also because air vortexes would be created inside the suction conduit, impairing its efficiency.

GB2334448A discloses an air suction cleaner for dentistry according to the preamble of claim 1.

A careful observation and a study of the aforesaid problem has led the applicant to devise the present invention in order to integrate a suction assembly in a dental unit or in any other structure comprising accessory units equipped with pneumatic and/or electronic devices.

More precisely, the purpose of the present invention is to devise an apparatus suitable for joining together, in a single frame, a suction assembly and an accessory unit having an electronic device, powered by electric cables and/or tubes, in such a way that the suction assembly and the accessory unit can be connected in a simple way to a single support element, such as, for example, a dental unit.

An additional purpose of the present invention is to disclose an apparatus that is inexpensive and easy to make.

These purposes are achieved according to the invention with the characteristics of the appended independent claim 1.

Advantageous embodiments appear from the dependent claims.

The apparatus according to the invention is defined by claim 1.

For the sake of clarity, the description of the apparatus according to the invention continues with reference to the appended drawings, which have a merely illustrative, not limiting value, wherein:
Fig. 1 is an axonometric view of an apparatus according to the invention suitable for being mounted on a ceiling;
Fig. 2 is an axonometric view of a portion of the apparatus of Fig. 1;
Fig. 3 is an axonometric view of a bearing frame of the apparatus according to the invention;
Fig. 3A is a cross-sectional view of the bearing frame shown in Fig. 3;
Fig. 4 and 5 are two axonometric views of a joint of the apparatus according to the invention, taken from a different angle, wherein said joint comprises only one secondary seat;
Fig. 4A is a side view of the joint of Fig. 4, sectioned along the plane A-A of Fig. 4;
Fig. 6 is a longitudinal view of the portion of the apparatus shown in Fig. 2;
Figs. 6A, 6B and 6C are the same views as Fig. 6, except for they show three different constructive variants of the apparatus according to the invention;
Fig. 7 is an axonometric view of the joint of the apparatus according to the invention in an alternative embodiment of the invention, wherein said joint comprises three peripheral compartments;
Fig. 8 is an axonometric view of an apparatus according to the invention comprising the joint of Fig. 7 and three accessory devices in addition to the suction device;
Fig. 9 is a diagrammatic axonometric view of a dental unit comprising the apparatus according to the invention.

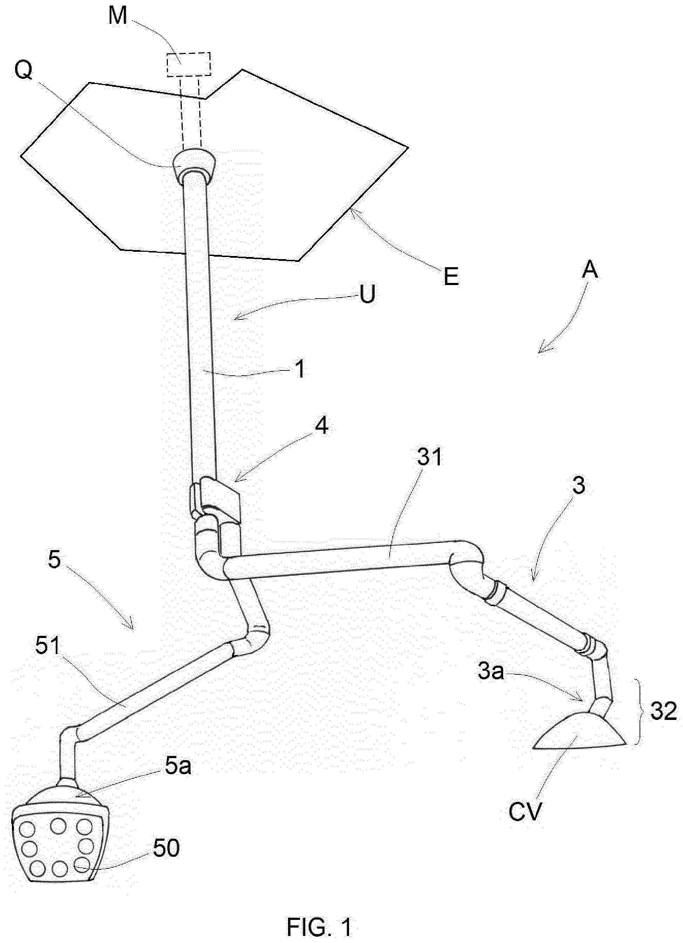

With reference to Figs. 1 to 8, an apparatus according to the invention is disclosed, which is generally indicated with reference letter A.

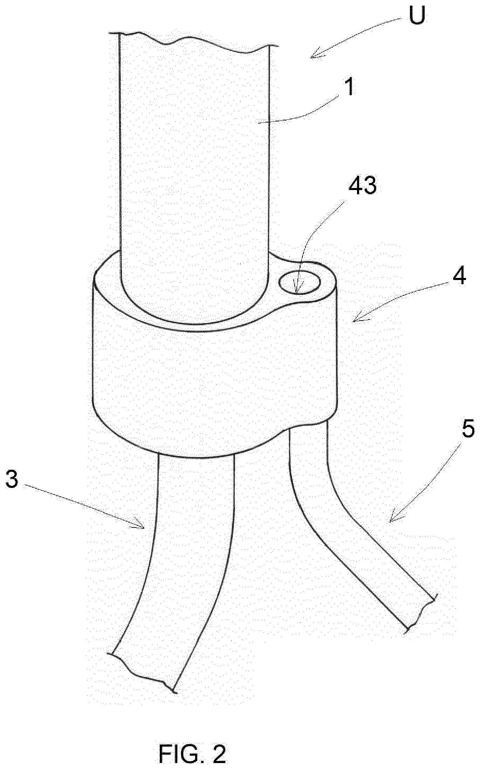

With reference to Figs. 1 and 2, in a first embodiment of the invention, said apparatus (A) comprises a bearing frame (U), a suction assembly (3), an accessory unit (5), a joint (4), and connection means (Q) for connecting the bearing frame (U) to a supporting element (E), such as a ceiling (as shown precisely in Fig. 1), or a floor, or a trolley or a frame of a dental unit.

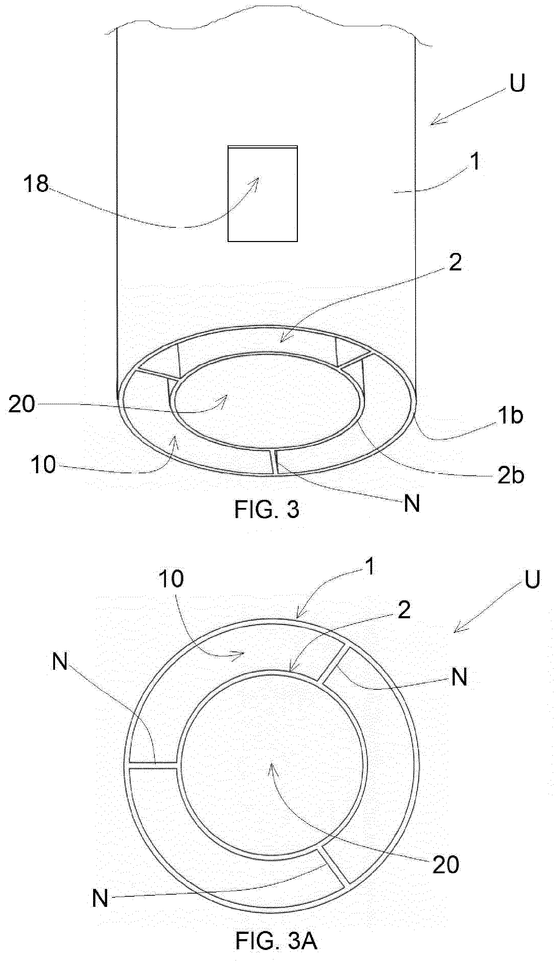

With reference to Fig. 3, the bearing frame (U) comprises a central tube (2) and an outer tube (1).

The central tube (2) is disposed coaxially inside the outer tube (1). Both the outer tube (1) and the central tube (2) have a circular section.

The bearing frame (U) comprises an annular peripheral duct (10) defined between the central tube and the external tube, and a central duct (20) defined by the central tube (2) and suitable for being in fluid communication with air suction means (M).

The air suction means (M) comprise, by way of example, helical aspirators, centrifugal aspirators, air ring aspirators, wet aspirators, liquid ring aspirators or Venture-type depression aspirators.

The outer tube (1) and the central tube (2) comprise a first end suitable for being connected to the supporting element (E) by means of the connection means (Q) disposed in correspondence with said first end, and a second end (1 b, 2b) opposite to said first end.

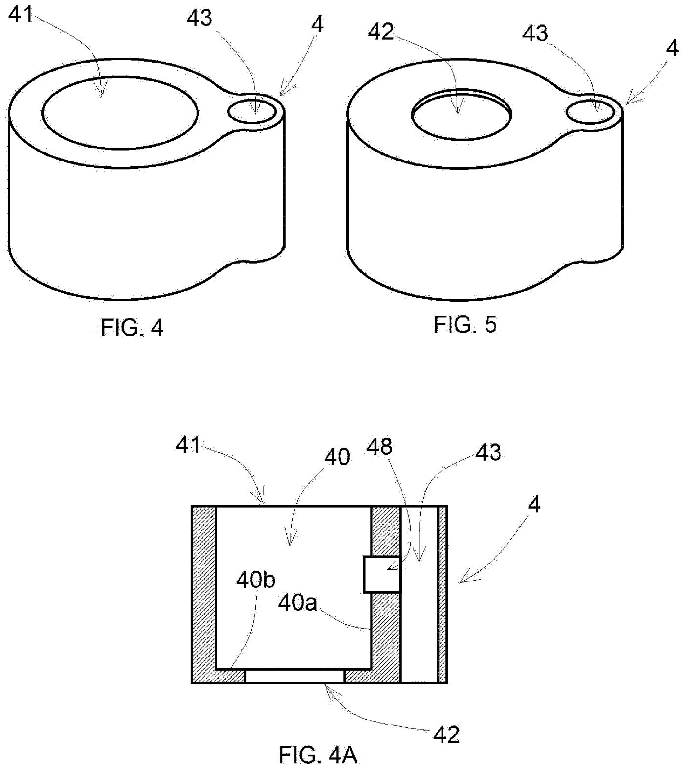

With reference to Figs. 4, 4A and 5, the joint (4) comprises a central compartment (40), a first inlet (41) for said central compartment (40) facing the connection means (Q), and a second inlet (42) facing the opposite side.

With reference to Fig. 4A, said joint (4) comprises a side surface (40a) that laterally defines the central compartment (40) and a bottom wall (40b) provided with the second mouth (42). The bottom wall (40b) of the joint comprises an inner side facing the central compartment (40) and an outer side facing the opposite side.

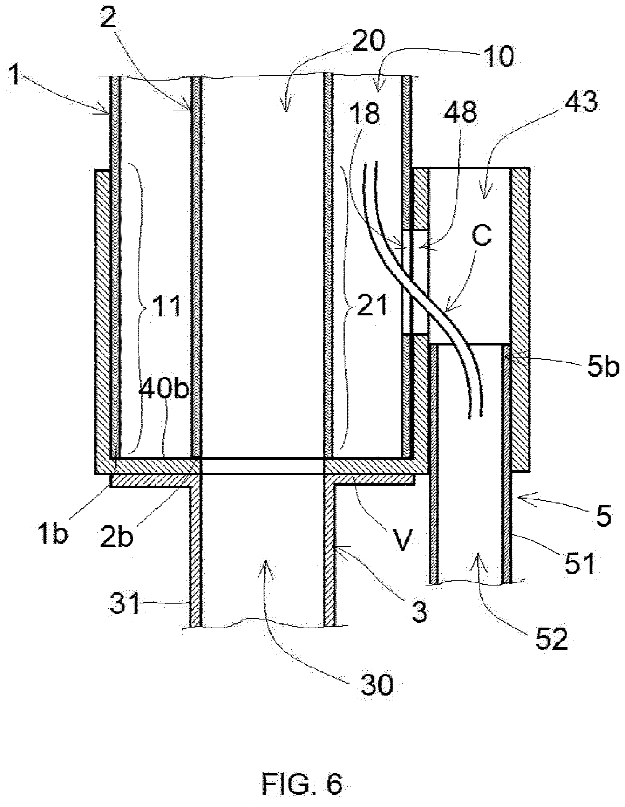

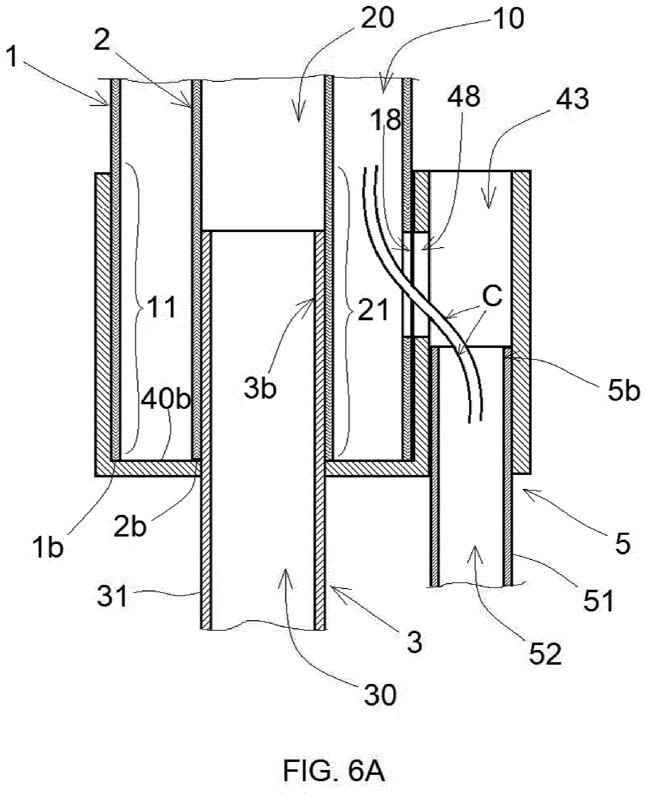

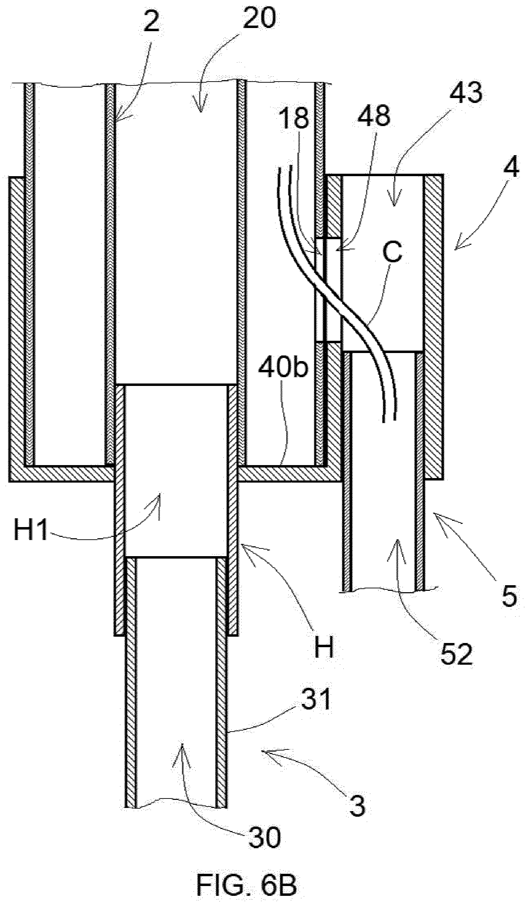

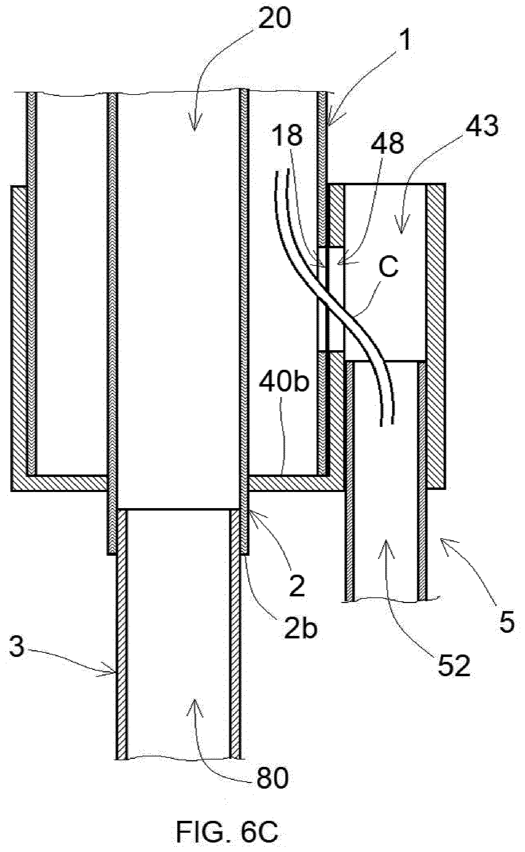

A section (21) of the central tube (2) is disposed inside the central compartment (40), as shown in Fig. 6.

The second end (2b) of the central tube (2) abuts the inner side of the bottom wall (40b) of the joint (4).

With reference to Figs. 1 and 6, the suction assembly (3) is installed downstream the central duct (20) and is in communication with it.

Said suction assembly (3) comprises a suction mouth (32) and a suction duct (30) used for sucking the air when the suction means (M) are activated.

Preferably, the suction duct (30) of the suction assembly (3) is disposed in an articulated and/or flexible supporting arm (31).

The supporting arm (31) has a first end (3a) comprising said suction mouth (32), and a second end (3b) fixed to the joint (4) in such a way that said suction duct (30) and said central duct (20) of the bearing frame are aligned and communicate with each other, as shown in Fig. 6.

The second end (3b) of the supporting arm (31) is provided with a flange (V) that is screwed onto the bottom wall (40b) of the joint (4), more specifically onto the outer side of the bottom wall (40b).

In an alternative embodiment of the invention, which is shown in Fig. 6A, the central duct (20) and the suction duct (30) of the supporting arm (31) can also be communicated with each other by inserting said second end (3b) of the supporting arm (31) in the central tube (2) in fit-in mode.

In an alternative embodiment of the invention, which is shown in Fig. 6B, a junction tube (H) is provided between the central tube (2) and the supporting arm (31) of the suction assembly (3) to connect the central tube (2) and the supporting arm (31). The junction tube (H) has an axial conduit (H1) that provides communication between the suction duct (30) and the central duct (20) of the central tube (2).

In an alternative embodiment of the invention, which is shown in Fig. 6C, said central tube (2) passes through the second inlet (42) of the joint and the second end (2b) projects out of the joint (4), instead of abutting the bottom wall (40b) of the joint (4).

Going back to Fig. 6, the outer tube (1) comprises an end section (11) housed in the central compartment (40) and has the second end (1b) abut the inner face of the bottom wall (40b) of the joint (4).

With reference to Figs. 4, 4A, 5 and 6, the joint (4) also comprises a peripheral compartment (43) disposed side by side with the central compartment (40).

The peripheral compartment (43) and the central compartment (40) communicate with each other.

An end of said bearing frame (U) is disposed in said central compartment (40) of the joint (4) so that said central duct (20) of the bearing frame (U) communicates with the suction duct (30) of the suction assembly (3) and said peripheral duct (10) of the bearing frame (U) communicates with said peripheral compartment (43) of the joint (4).

The joint (4) has an opening (48) that puts the central compartment (40) in communication with the peripheral compartment (43).

The outer tube (1) of the bearing frame (U) has an opening (18) disposed in correspondence with the opening (48) of the joint (4).

With reference to Figs. 1 and 6, an accessory unit (5) comprises an articulated and/or flexible supporting arm (51) comprising:
- a duct (52),
- a first end (5a) provided with a pneumatic and/or electric/electronic accessory (50); and
- a second end (5b) connected to the joint (4) in such a way that the due (52) communicates with the peripheral compartment (43) of the joint; in particular, the second end (5b) of the supporting arm (51) is inserted in the peripheral compartment (43) of the joint.

With reference to Fig. 6, the pneumatic and/or electric/electronic accessory (50) comprises tubes and/or electrical cables (c) that pass through the duct (52) of the supporting arm of the accessory unit, the peripheral compartment (43) of the joint (4), and the peripheral duct (10) of the bearing frame.

By way of example, the pneumatic and/or electric/electronic accessory (50) may consist of a lamp, a monitor, a tray or bottle holder with spray feed, a micro-sandblaster, an intra/extraoral camera, and the like.

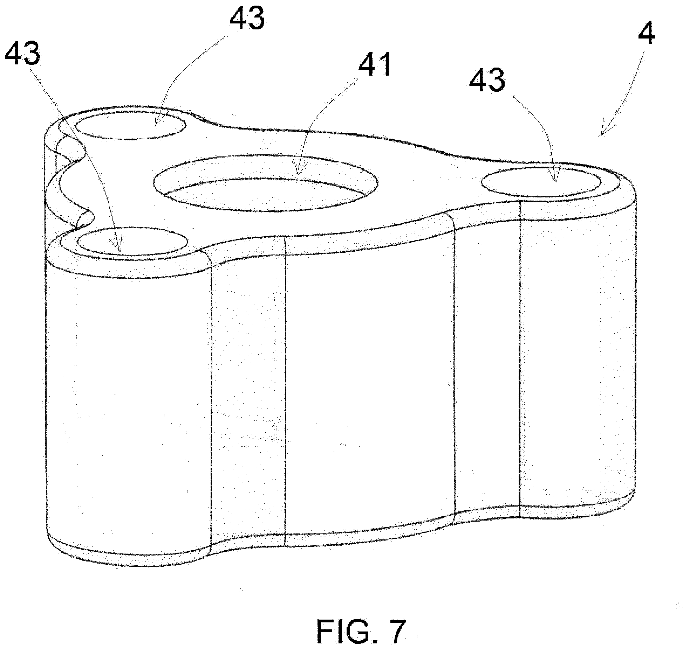

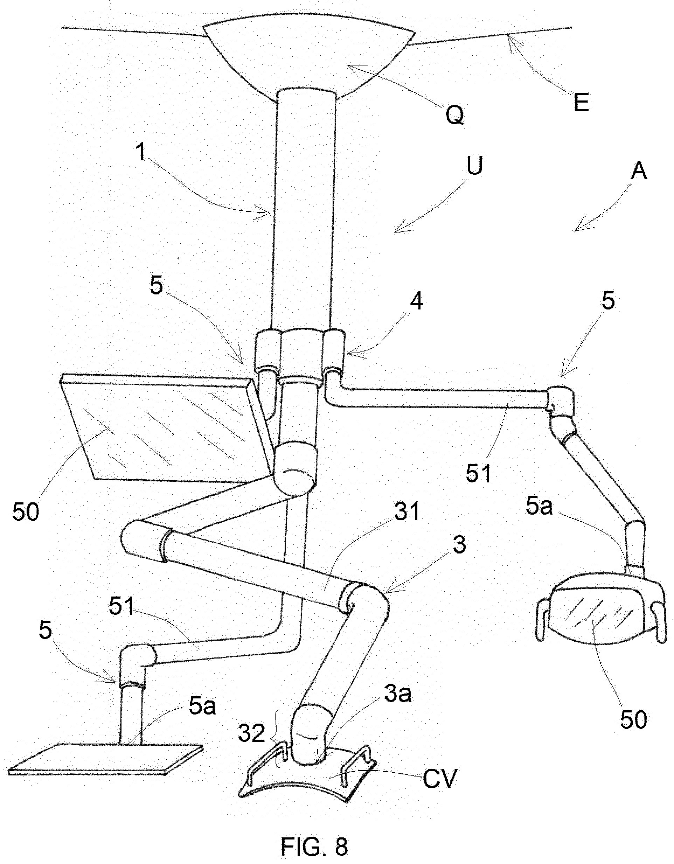

With reference to Figs. 7 and 8, in an alternative embodiment of the invention, the apparatus (A) may comprise a plurality of peripheral compartments (43) disposed around the central compartment (40), equally spaced angularly from each other, and as many accessory units (5).

In such a case, openings (48, 18) in the same number as the number of peripheral compartments (43) are obtained on the joint (4) and on the end section (11) of the outer tube (1).

Going back to Figs. 3 and 3A, preferably, in the case when multiple accessory units (5) and multiple peripheral compartments (43) are provided, said bearing frame (U) comprises a plurality of radial partitions (N) disposed in the peripheral duct (10) extending for the entire length of the outer tube (1) in such a way to define multiple passage portions for the tubes and/or electric cables (c). Each passage portion is associated with one of said peripheral compartments (43).

Optionally, the suction assembly (3) comprises a spout or conveyor hood (CV) disposed in the suction mouth (32).

Optionally, the suction assembly (3) may comprise LEDs or lamps disposed in proximity to said suction mouth (32) and powered by a battery.

Furthermore, although not shown in the appended figures, the suction assembly (3) may comprise adjustment means for adjusting the width of the suction mouth (32).

By way of example, said adjustment means comprise a movable partition disposed in correspondence with the suction mouth (32) in such a way that the movable partition can be moved between:
- a closing position, wherein the inlet is completely covered and closed by the movable partition; and
- an opening position, wherein the inlet is completely free.

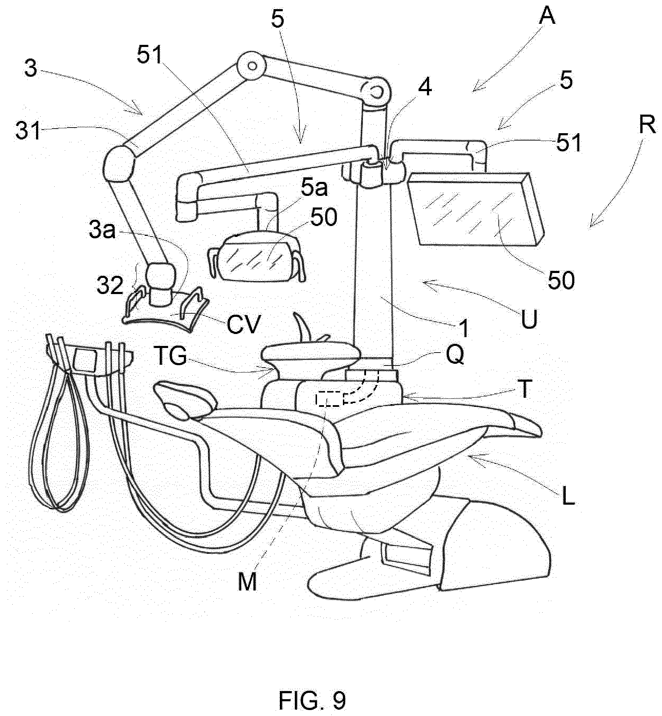

With reference to Fig. 9, a further object of the present invention is a dental unit (R) comprising the apparatus (A) according to the invention.

The dental unit (R) comprises:
- a bed (L);
- a supporting frame (T) of a water system (TG); said frame (T) being fixed or integral with the bed (L);
- the apparatus (A) as described above; and
- connection means (Q) for connecting the bearing frame (U) of the apparatus (A) and the supporting frame (T) of the water system (TG).

It should be noted that although in the appended figures the outer tube (1) is always shown around the central tube (2), the outer tube could be parallel to the central tube.

Even if it has always been assumed that the apparatus (A) in question is used in dental practices, said apparatus (A) can be used for different purposes, such as in surgical rooms or generic "workstations", for example in beauty and aesthetics centers, podiatry, veterinary, tattoos, and wherever it is necessary to provide a suction system and various accessories, such as illumination of the working field or magnifiers, supporting tablets, monitors with integrated PC. Moreover, the apparatus according to the invention can be used also in other sectors, such as the chemical pharmaceutical industry, in work stations used for welding or finishing operations of materials.

In view of the above, evidently, because of the particular configuration of the joint (4), the new apparatus (A) according to the invention can integrate the suction assembly and the accessory units in a single bearing frame (U) connected to a supporting element, such as a dental unit, while maintaining the tubes and/or electric cables of the electric/electronic accessories of the accessory units.

## Claims

1. Apparatus (A) comprising:
- a bearing frame (U) suitable for being connected to a supporting element (E, T) and to suction means (M);
- a suction assembly (3) connected to the bearing frame (U); said suction assembly (3) comprising a suction mouth (32) connected to a suction duct (30) in order to suck the air;
- a joint (4) connecting the bearing frame (U) to the suction assembly (3);
- at least one accessory unit (5) comprising a pneumatic and/or electric/electronic accessory (50) and a supporting arm (51) with a duct (52) connected to the joint (4),
wherein said bearing frame (U) comprises a central duct (20);
said joint (4) comprises a central compartment (40),
wherein an end of said bearing frame (U) is disposed in said central compartment of the joint (4) so that said central duct (20) of the bearing frame (U) communicates with the suction duct (30) of the suction assembly (3),
**characterized in that**
said bearing frame (U) comprises a peripheral duct (10);
said joint (4) comprises and a peripheral compartment (43) in communication with said central compartment (40);
an end of said duct (52) of the supporting arm (51) of the accessory unit (5) is arranged in said peripheral compartment (43);
said peripheral duct (10) of the bearing frame (U) communicates with said peripheral compartment (43) of the joint (4) and with said duct (52) of the supporting arm of the accessory unit (5).

2. The apparatus (A) of claim 1, wherein said bearing frame (U) comprises a central tube (2) that defines said central duct (20) and an outer tube (1) so that said peripheral duct (10) is defined between the central tube (2) and the outer tube (1).

3. The apparatus (A) of claim 1 or 2, comprising suction means (M) in communication with the central duct (20).

4. The apparatus (A) according to any one of the preceding claims, wherein said suction duct (30) of the suction assembly (3) is disposed inside an articulated and/or flexible supporting arm (31).

5. The apparatus (A) according to any one of claims 2 to 4, wherein said joint (4) comprises an opening (48) that puts the central compartment (40) in communication with the peripheral compartment (43); and
the outer tube (1) of the bearing frame (U) comprises an opening (18) disposed in correspondence with said opening (48) of the joint (4).

6. The apparatus (A) according to any one of the preceding claims, wherein said joint (4) comprises a lateral surface (40a) that defines said central compartment (40) laterally, and a bottom wall (40b) provided with a mouth (42) in communication with the suction duct (30); wherein an end of the bearing frame (U) abuts said bottom wall (40b) of the joint (4).

7. The apparatus (A) of claim 6 when depending on claim 4, wherein said central duct (20) of the central tube (2) and said suction duct (30) are aligned.

8. The apparatus (A) according to any one of the preceding claims, wherein said joint (4) comprises a plurality of peripheral compartments (43) disposed around the central compartment (40) that are equally angularly spaced, and said apparatus (A) comprises a plurality of accessory units (5) connected to the peripheral compartments (43) of the joint (4).

9. The apparatus (A) of claim 8, wherein said bearing frame (U) comprises a plurality of radial partitions (N) that are disposed in the peripheral duct (10), extending for the entire length of the outer tube (1) in such a way to define multiple passage portions for tubes and/or electric cables.

10. Dental unit (R) comprising:
- a bed (L);
- a supporting frame (T) of a water system (TG) fixed to the floor or integral with said bed (L);
- an apparatus (A) according to any one of the preceding claims; and
- connection means (Q) for connecting the bearing frame (U) of the apparatus (A) to the supporting frame (T) of the water system (TG).

## Patentansprüche

1. Ausrüstung (A), umfassend:
- ein tragendes Gestell (U), das dazu bestimmt ist, mit einem Stützelement (E; T) und Saugmitteln (M) verbunden zu werden;
- eine Sauganordnung (3), die mit dem tragenden Gestell (U) verbunden ist; wobei die Sauganordnung (3) eine Saugmündung (32) umfasst, die mit einer Saugleitung (30) verbunden ist, um Luft anzusaugen;
- ein Verbindungsstück (4), welches das tragende Gestell (U) mit der Sauganordnung (3) verbindet,
- mindestens ein Zusatzgerät (5), das ein pneumatisches und/oder elektrisches/elektronisches Zusatzteil (50) und einen Tragarm (51) mit einer Leitung (52) umfasst, die mit dem Verbindungsstück (4) verbunden ist,
wobei
das tragende Gestell (U) eine zentrale Leitung (20) umfasst;
das Verbindungsstück (4) ein zentrales Fach (40) umfasst,
ein Endabschnitt des tragenden Gestells (U) in dem zentralen Fach des Verbindungsstücks (4) angeordnet ist, so dass die zentrale Leitung (20) des tragenden Gestells (U) mit der Saugleitung (30) der Sauganordnung (3) in Verbindung steht,
**dadurch gekennzeichnet, dass**
das tragende Gestell (U) eine periphere Leitung (10) umfasst,
das Verbindungsstück (4) ein peripheres Fach (43) umfasst, das mit dem zentralen Fach (40) in Verbindung steht,
ein Endabschnitt der Leitung (52) des Tragarms (51) des Zusatzgeräts (5) in dem peripheren Fach (43) angeordnet ist,
die periphere Leitung (10) des tragenden Gestells (U) mit dem peripheren Fach (43) des Verbindungsstücks (4) und mit der Leitung (52) des Tragarms des Zusatzgeräts (5) in Verbindung steht.

2. Ausrüstung (A) nach Anspruch 1, wobei das tragende Gestell (U) ein Zentralrohr (2), das die zentrale Leitung (20) definiert, und ein Außenrohr (1) umfasst, so dass die periphere Leitung (10) zwischen dem Zentralrohr (2) und dem Außenrohr (1) definiert ist.

3. Ausrüstung (A) nach Anspruch 1 oder 2, umfassend Saugmittel (M), die mit der zentralen Leitung (20) in Verbindung stehen.

4. Ausrüstung (A) nach einem der vorstehenden Ansprüche, wobei die Saugleitung (30) der Sauganordnung (3) im Innern eines gelenkigen und/oder flexiblen Tragarms (31) angeordnet ist.

5. Ausrüstung (A) nach einem der Ansprüche 2 bis 4, wobei das Verbindungsstück (4) eine Öffnung (48) aufweist, die das zentrale Fach (40) mit dem peripheren Fach (43) in Verbindung bringt; und
das Außenrohr (1) des tragenden Gestells (U) eine Öffnung (18) aufweist, die in Übereinstimmung mit der Öffnung (48) des Verbindungsstücks (4) angeordnet ist.

6. Ausrüstung (A) nach einem der vorstehenden Ansprüche, wobei das Verbindungsstück (4) eine seitliche Oberfläche (40a), die das zentrale Fach (40) seitlich definiert, und eine Bodenwand (40b) umfasst, die mit einer Mündung (42) versehen ist, die mit der Saugleitung (30) in Verbindung steht; wobei ein Endabschnitt des tragenden Gestells (U) gegen die Bodenwand (40b) des Verbindungsstücks (4) in Anschlag geht.

7. Ausrüstung (A) nach Anspruch 6, sofern abhängig von Anspruch 4, wobei die zentrale Leitung (20) des Zentralrohrs (2) und die Saugleitung (30) aufeinander ausgerichtet sind.

8. Ausrüstung (A) nach einem der vorstehenden Ansprüche, wobei das Verbindungsstück (4) eine Vielzahl von peripheren Fächern (43) umfasst, die um das zentrale Fach (40) herum angeordnet sind und gleichmäßig voneinander beabstandet sind, und wobei die Ausrüstung (A) eine Vielzahl von Zusatzgeräten (5) umfasst, die mit den peripheren Fächern (43) des Verbindungsstücks (4) verbunden sind.

9. Ausrüstung (A) nach Anspruch 8, wobei das tragende Gestell (U) eine Vielzahl von radialen Zwischenwänden (N) umfasst, die in der peripheren Leitung (10) angeordnet sind und sich über die gesamte Länge des Außenrohrs (1) erstrecken, derart, dass mehrere Durchgangsabschnitte für Rohre und/oder Elektrokabel definiert werden.

10. Zahnärztliche Behandlungseinheit (R), umfassend:
- einen Behandlungsstuhl (L);
- ein Gestell (T) einer Wassereinheit (TG), die am Boden befestigt oder fest mit dem Behandlungsstuhl (L) verbunden ist;
- eine Ausrüstung (A) nach einem der vorstehenden Ansprüche; und
- Verbindungsmittel (Q) für die Verbindung des tragenden Gestells (U) der Ausrüstung (A) am Gestell (T) der Wassereinheit (TG).

## Revendications

1. Appareil (A) comprenant :
- un châssis de support (U) destiné à être relié à un élément de support (E, T) et à des moyens d'aspiration (M) ;
- un groupe d'aspiration (3) relié au châssis de support (U) ; ledit groupe d'aspiration (3) comprenant une embouchure d'aspiration (32) reliée à un conduit d'aspiration (30) pour aspirer l'air ;
- un joint (4) qui relie le châssis de support (U) au groupe d'aspiration (3) ;
- au moins un groupe accessoires (5) comprenant un accessoire pneumatique et/ou électrique/électronique (50) et un bras de support (51) ayant un conduit (52) relié au joint (4),
où
ledit châssis de support (U) comprend un conduit central (20) ;
ledit joint (4) comprend un compartiment central (40),
où une extrémité du ledit châssis de support (U) est disposée dans ledit compartiment central du joint (4), de manière que ledit conduit central (20) du châssis de support (U) communique avec le conduit d'aspiration (30) du groupe d'aspiration (3),
**caractérisé en ce que**
ledit châssis de support (U) comprend un conduit périphérique (10),
ledit joint (4) comprend un compartiment périphérique (43) qui communique avec ledit compartiment central (40),
une extrémité dudit conduit (52) du bras de support (51) du groupe accessoires (5) est disposée dans ledit compartiment périphérique (43),
ledit conduit périphérique (10) du châssis de support (U) communique avec ledit compartiment périphérique (43) du joint (4) et avec ledit conduit (52) du bras de support du groupe accessoires (5).

2. Appareil (A) selon la revendication 1, où ledit châssis de support (U) comprend un tube central (2) qui définit ledit conduit central (20) et un tube externe (1), de manière à ce que ledit conduit périphérique (10) soit défini entre le tube central (2) et le tube externe (1).

3. Appareil (A) selon la revendication 1 ou 2, comprenant des moyens d'aspiration (M) qui communiquent avec le conduit central (20).

4. Appareil (A) selon l'une quelconque des revendications précédentes, où ledit conduit d'aspiration (30) du groupe d'aspiration (3) est disposé dans un bras de support (31) articulé et/ou flexible.

5. Appareil (A) selon l'une quelconque des revendications de 2 à 4, où ledit joint (4) a une ouverture (48) qui met en communication le compartiment central (40) avec le compartiment périphérique (43) ; et
le tube externe (1) du châssis de support (U) a une ouverture (18) disposée en correspondance de ladite ouverture (48) du joint (4).

6. Appareil (A) selon l'une quelconque des revendications précédentes, où ledit joint (4) comprend une surface latérale (40a) qui délimite latéralement ledit compartiment central (40), et une paroi de fond (40b) sur laquelle est réalisée une embouchure (42) qui communique avec le conduit d'aspiration (30) ; où une extrémité du châssis de support (U) est en butée contre ladite paroi de fond (40b) du joint (4).

7. Appareil (A) selon la revendication 6 quand dépendante de la revendication 4, où ledit conduit central (20) du tube central (2) et ledit conduit d'aspiration (30) sont alignés entre eux.

8. Appareil (A) selon l'une quelconque des revendications précédentes, où ledit joint (4) comprend une pluralité de compartiments périphériques (43) disposés autour du compartiment central (40), angulairement éloignés de manière égale entre eux, et ledit appareil (A) comprend une pluralité de groupes accessoires (5) reliés aux compartiments périphériques (43) du joint (4).

9. Appareil (A) selon la revendication 8, où ledit châssis de support (U) comprend une pluralité de cloisons radiales (N), disposées dans le conduit périphérique (10), qui se déploient sur toute la longueur du tube externe (1) afin de délimiter plusieurs portions de passage pour des tubes et/ou des câbles électriques.

10. Unité dentaire (R) comprenant :
- un fauteuil dentaire (L) ;
- un châssis (T) d'un groupe hydrique (TG) fixé au sol ou solidaire avec ledit fauteuil dentaire (L) ;
- un appareil (A) selon l'une quelconque des revendications précédentes ; et
- des moyens de connexion (Q) pour relier le châssis de support (U) de l'appareil (A) au châssis de support (T) du groupe hydrique (TG).
